Europäisches Patentamt

**European Patent Office**

Office européen des brevets

(19)

(11) Publication number: **0 050 473**
A2

# (12) EUROPEAN PATENT APPLICATION

(21) Application number: **81304790.9**

(22) Date of filing: **14.10.81**

(51) Int. Cl.³: **F 25 B 21/02, A 42 B 3/00, A 41 B 13/00, B 60 H 3/00**

(30) Priority: **14.10.80 US 196599**

(43) Date of publication of application: **28.04.82 Bulletin 82/17**

(84) Designated Contracting States: **DE FR GB IT**

(71) Applicant: **INVENTORS & INVESTORS, INC., 78 Woodlawn Drive, Williamstown Massachusetts 01267 (US)**

(72) Inventor: **Lehovec, Kurt, 202 S Juanita Avenue Apartment 2-214, Los Angeles California 90004 (US)**
Inventor: **Shuttleworth, Rose Allen, 1522 First Street Apartment M 204, Coronado California 92118 (US)**
Inventor: **Bedri, Yussef A., Deceased, Riyadh (SA)**

(74) Representative: **Barrett, James William et al, A.A. THORNTON & CO. Northumberland House 303-306 High Holborn, London WC1V 7LE (GB)**

(54) **Personalized cooling systems.**

(57) This invention provides various personalized cooling systems in which heat relief is achieved by Peltier cooling. In one embodiment Peltier cells are attached to a garment with the cold plate of the Peltier cell in intimate thermal contact with the skin of the wearer of the garment. Heat generated by the Peltier cell is dissipated to the ambient from cooling fins. Heat pipes are used to conduct the heat to the fins, or to distribute the cooling across the skin.

In another embodiment, personalized cooling in a closed environment such as a room, tent, or car is provided by selective Peltier cooling of garments or accessories in close thermal contact with the person to be cooled. The Peltier cells are powered from stationary power sources, where available, such as room electrical outlets or car batteries. In other embodiments the Peltier cells are powered by portable rechargeable batteries, or by solar cells carried by the person to be cooled. The rechargeable batteries are charged by portable solar cells.

- 1 -
Personalized Cooling Systems

This invention relates to personalized cooling systems.

There is ample need for heat relief for humans in a hot ambient. In some cases heat relief provides merely comfort, e.g. in home, office, or car air conditioning. In other cases heat relief is a necessity for proper functioning of the human body, e.g. during re-entry of astronauts, during mine worker's rescue missions, and for fighter pilots in confined cockpits.

The metabolic rate of a human at rest is about 100 Watts, and it increases to about 300 Watts under a heavy work load. This additional energy generation rate is being dissipated to the environment mainly by the evaporation of perspiration. Excessive sweating leads to dehydration and loss of salts from the body, causing exhaustion.

In severe cases heat relief has been provided by specially designed garments containing a circulating cooling liquid, viz., "Water Cooled Garments: A Review" by Sarah A. Nunneley, published in Space Life Sciences, 2 (1970) 335-360.

In less severe cases of merely providing comfort, such as in room air conditioning, there is an urgent need to conserve energy.

Tourists in hot countries, and outdoor manual workers in a hot ambient, e.g. construction workers in desert areas, would greatly benefit by personalized cooling means for comfort and increased work·efficiency. The liquid cooled garments which are commercially available, e.g. from 1LC Dover Corporation, Frederica, Delaware, are clearly unsuitable for these applications.

The present energy shortage requires energy savings. A major energy consumer is the air conditioning of entire enclosures, such as rooms, offices, and automobiles. Only a small fraction of this cooling is consumed by the inhabitants. By providing personalized cooling very significant energy savings could be achieved.

It is an objective of this invention to provide a simple personalized cooling means.

It is another objective of this invention to provide a convenient garment providing cooling to the wearer.

It is still another objective of this invention to provide a cooling garment powered by a portable power source affixed to the garment of the wearer.

It is still another objective of this invention to provide a cooling garment powered by solar cells carried by the wearer.

It is still another objective of this invention to provide a cooling garment powered by portable rechargeable batteries in combination with transportable solar power means to recharge these batteries.

It is still another objective of this invention to provide heat relief in confined space by directly cooling the person in this space, rather than the entire space, thereby saving energy.

It is still another objective of this invention to provide a cooling garment or accessory which can be transformed into a heating garment or accessory by reversing the polarity of the current which activates said garment or accessory.

The present invention provides personalized cooling systems for warm blooded species, man or animal, by thermal contact with the cold plate of one or more Peltier cells. In one embodiment of the invention the Peltier cell or cells are attached to a garment in such a way that the cold plate of the Peltier cell or cells contacts the skin of the wearer, while the heat generated by the Peltier cell is dissipated by means of cooling fins in good thermal contact with the hot plate of the Peltier cells. In a preferred embodiment heat pipes are used for the transport of heat from the hot plates to the cooling fins. A preferred location for cooling fins is the exterior of a helmet such as is worn by construction workers or motorcyclists. In another preferred embodiment, a flat curved heat pipe is used to distribute the low temperature of the cold plate of the Peltier cell over a larger skin area. The preferred garments of this invention are head bands and neck bands carrying the Peltier cells.

In a preferred embodiment the Peltier cell or cells are powered by solar batteries mounted on the brim of a wide-brimmed hat, thus providing a self-powered portable unit. In another embodiment rechargeable batteries are used for powering the Peltier cells, with batteries attached to a belt, or else to the aforementioned construction workers' helmet. A portable rack of solar cells is provided to recharge the batteries.

In a closed environment such as an office, room, car, or tent, personalized cooling is provided by Peltier cooled garments, or by Peltier cooled furnishing such as chairs, or sleeping bags. The Peltier cells can be powered from standard electric outlets where available, with suitable means for transferring the a.c. voltage of the outlet to the low d.c. voltage required by the Peltier cell. The car battery power can be used in automobiles; however, solar cell arrays carried on the upper surface of the car can also be used, which is desirable for parked cars. Personalized Peltier cooling in a tent is powered by a solar cell rack along one face of the tent.

Reversal of the polarity of the current passed through a Peltier cell exchanges the hot and cold plates, thus providing heating for the skin, while absorbing cooling from the ambient by the fins. Thus the aforementioned cooling garments or fixtures can be transformed into heating garments or fixtures.

Embodiments of the invention will now be described, by way of example only, with reference to the accompanying drawings in which:-

Figure 1 illustrates a conventional Peltier cell;

Figure 2 shows a broad-brimmed hat carrying a solar cell array according to this invention, and a head band carrying three Peltier cells powered by said solar cell array;

Figure 3 shows a top view of a Peltier cooled collar according to this invention for cooling forehead or rear of the neck, in combination with a belt carrying a battery pack for supplying power to the Peltier cell;

Figure 4 illustrates a foldable solar cell rack for charging rechargeable batteries as used to power the Peltier cell in Figure 3;

Figure 5 shows a construction workers' helmet according to this invention, carrying heat dissipation fins connected by heat pipes to the hot plates of Peltier cells cooling forehead and neck of the wearer;

Figure 6 illustrates a Peltier cooled chair according to this invention for personalized cooling in a room, with means to power the Peltier cells in said chair from a room outlet over a transformer and rectifier box;

Figure 7 shows an automobile with driver's seat cooled by Peltier cells connected by a heat pipe to cooling fins at the outside of the automobile. Also shown are cooling means for the forehead and rear of the neck of the driver, which are suspended from the car top, and are powered by a solar cell array mounted on the car roof, according to this invention, and

Figure 8 shows a tent according to this invention, faced on one side with a solar cell array which provides electric power for personalized cooling means inside the tent.

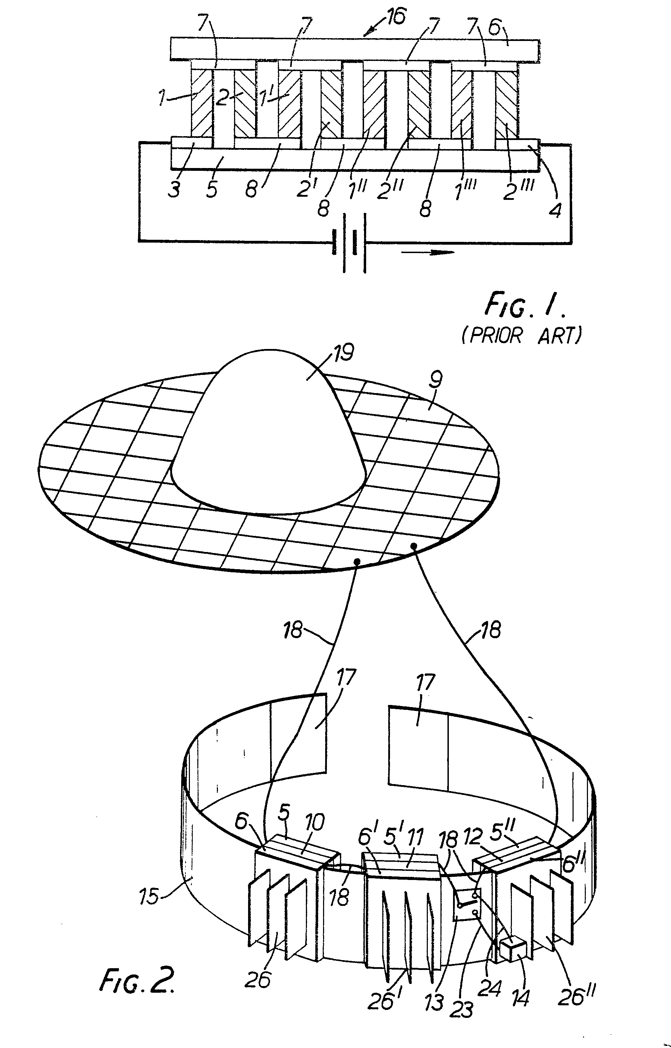

Referring to Figure 1, there is shown a cross section of a Peltier cell 16 as is commercially available from several sources, e.g. Borg Warner Thermoelectrics, Chicago, Illinois; or Cambridge Thermionic Corporation, Cambridge, Massachusetts. The Peltier cell comprises several columns 1, 1', etc., of an n-type semiconductor, and several columns 2, 2', etc., of a p-type semiconductor, interconnected by contacts 7 and 8. Direct current passed through the external contacts 3 and 4, with 4 positive, and 3 negative, heats the upper junctions 7 where the current flows from p- to n-type material, while it cools the lower junctions 8 where the current flows from n- to p-type material. Thus the upper insulator plate 6 becomes hot, while the lower insulator plate 5 is cooled.

The invention provides for cooling of the body by means of Peltier cells in intimate thermal contact with portions of the body such that the cold plate faces the skin, and the hot plate faces the ambient. The skin temperature must not be lowered to less than about $17^{o}C$, in order to avoid cold pain. Furthermore, heat conduction from the core of the body to the skin occurs primarily

- 6 -

through the blood stream, and this heat transfer is decreased by cardiovascular constriction when the skin temperature is too low. For a Peltier cell of about 6.45 square centimetres (1 square inch) area, such as the Model 930-71 supplied by Borg Warner Thermoelectrics, at a current of the order of 1 ampere and a voltage of about 2 volts, the temperature difference between the hot and cold plates is about $20^{\circ}C$ at a cooling rate of 4 Watts and a hot plate temperature of about $40^{\circ}C$.

The Peltier cells are mounted on garments, or in fixtures, as will be illustrated in the following preferred embodiments.

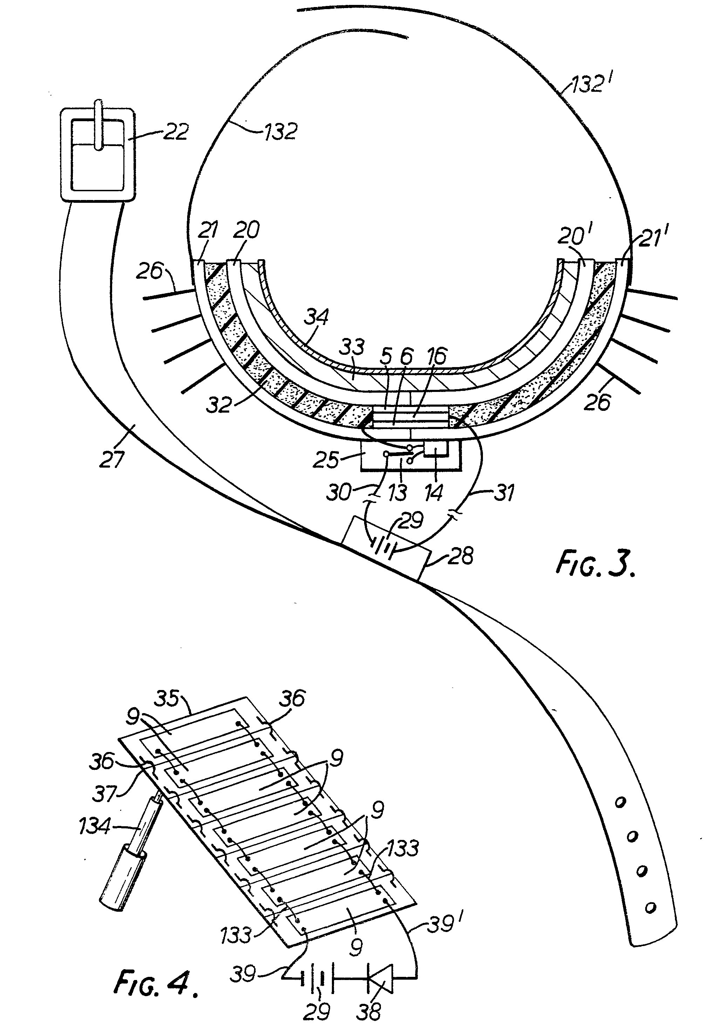

Referring now to Figure 2, there is shown an unfolded band 15 containing the Peltier cells 10, 11 and 12. The hot plates 6, 6', and 6" of these Peltier cells face outward and carry the heat fins 26, 26', and 26", while the cold plates 5, 5', and 5" face inside to the forehead when the headband 15 is fastened around the head using the adjustable Velcro fastener 17. The Peltier cells are powered through leads 18 by the solar cell array 9 located on the wide brim of hat 19. The electric connection between the solar cell array and the Peltier cells includes the switch 13 mounted on the headband which allows inserting into the circuit through leads 23 and 24 the thermistor 14 which is mounted in close thermal contact with the heat dissipation fin 26". The thermistor has the following purpose: if the power to the Peltier cell were suddenly interrupted, heat from the hot plate would quickly transfer to the cold plate, causing a burning sensation on the skin. By inserting the thermistor 14 into the circuit, rather than interrupting the circuit, the current through the Peltier cells is reduced by the thermistor resistance, which reduces the cooling rate and the power dissipation in the Peltier cells, and thus the hot plate temperature. This in turn reduces the thermistor temperature, whose resistance thereby increases, causing further reduction in current, cooling rate, heat fin and thermistor temperatures, etc. In this manner a gradual cooling of the hot plate temperature and a gradual warming of the cold plate temperature can be achieved by appropriate choice of heat capacity, heat dissipation rate from the cooling fins, and thermistor characteristics. Electric power of 7 Watts used to operate the three Peltier cells is readily provided by silicon solar cells mounted on an eight inch wide brim, at noon time solar radiation. While the cooling rate of about 15 Watts achieved with three Borg Warner Model 930-71 Peltier cells under such operating conditions is only a small fraction of the metabolic rate at rest, we have, nevertheless, experienced a significant degree of comfort by this forehead cooling.

A considerably larger solar cell array can be accommodated by a stationary structure such as a beach umbrella. Such a solar cell array can thus power more Peltier cells, arranged, e.g., on a head and a neck band.

Figure 3 shows a top view of another preferred embodiment for a head or neck cooling collar, providing Peltier cooling. In this embodiment a single Peltier cell 16 is arranged in such a manner that its cold plate 5 connects to the bent flat heat pipes 20, 20', while its hot plate 6 connects to the bent flat heat pipes 21 and 21'. The heat pipes 20 and 20' are curved to fit the forehead or the neck, as the case may be. The space between the heat pipes is filled with a material 32 of low heat conductance, while the concave surface of the heat pipes 20 and 20' is lined with a resilient, thermally conducting layer 33. This resilient material can be made from foam rubber vacuum impregnated with heat conducting grease. A metallized cloth layer 34 which lines the resilient material 33 contacts the skin when the garment is worn. The box 25 contains the switch 13 and thermistor 14 combination discussed in connection with Figure 2. The heat fins 26 dissipate the heat from the outer heat pipes 21 and 21'. The belt 27 with buckle 22 carries the compartment 28 which houses a rechargeable battery 29 for powering the Peltier cell 16 through wires 30 and 31. The bands 132, 132' are used to attach the Peltier cooling arrangement of Figure 3 to the forehead or to the rear of the neck, as the case may be. The single Peltier cell 16 in Figure 3 is operated at substantially higher current levels, i.e., several amperes, than are the cells 10, 11 or 12 in Figure 2, yet the heat pipes 21, 21' keep the hot plate temperature at about $35^{\circ}C$ at an ambient temperature of $25^{\circ}C$.

Figure 4 shows a rack 35, carrying solar cells 9, supported by a collapsible shaft 134, with hinges 36 which permit the rack to be folded along the lines 37. The folded solar cell rack fits into an attache case for ease of transport. The solar cell rack 35 charges the battery 29 of Figure 3 when discharged, through leads 39 and 39' over rectifier 38. The leads 133 connect the solar cells in the various sections of rack 35.

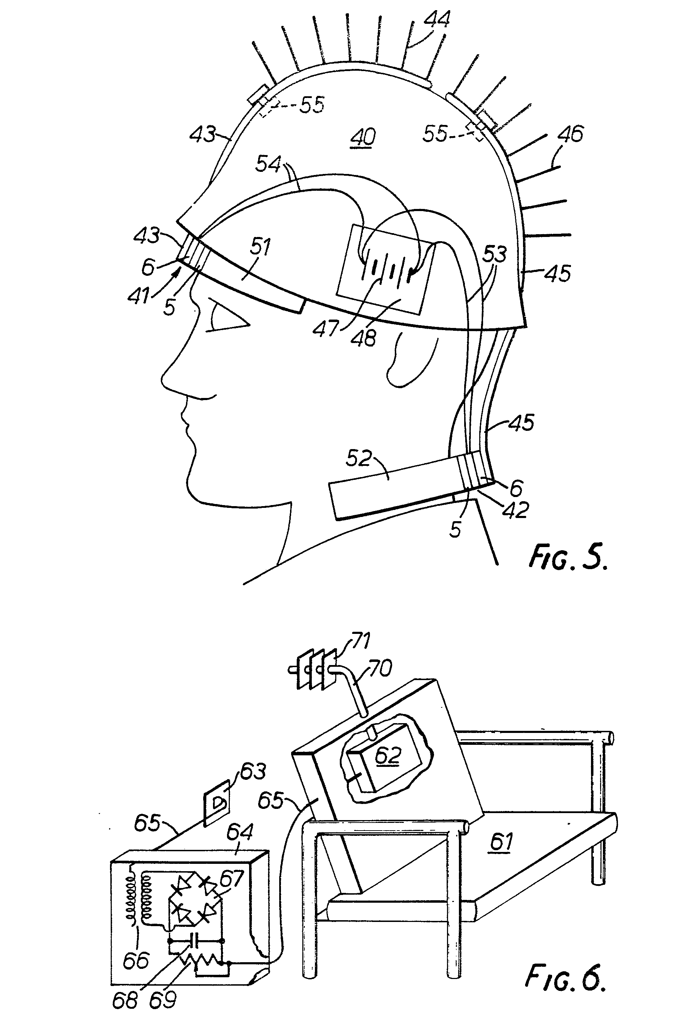

Figure 5 shows another preferred embodiment of the invention, in which a construction workers' helmet 40 is used as support for the Peltier cells 41 and 42. The cold plates 5 of these Peltier cells connect to curved flat heat pipes 51 and 52 to provide cooled forehead and neck collars similar to that shown in Figure 3. The heat pipes 51 and 52 distribute the cooling action of the Peltier cells 41 and 42 over a larger skin area. The heat from the hot plate 6 of the Peltier cell 41 which cools the forehead is carried by the heat pipe 43 to the heat dissipating fins 44 on the top of the helmet. Similarly, the heat pipe 45 conducts the heat from the hot plate 6 of the Peltier cell 42 which cools the neck to the heat

- 9 -

fins 46 on the top of the helmet. The heat pipe 45 is shaped to press the cooled collar 52 against the neck. The heat pipes 43 and 45 are attached to the helmet 40 by clamps 55. The Peltier cells are powered through leads 53 and 54 by a battery pack 47 contained in a compartment 48 of the helmet.

Figure 6 shows a chair 61, assumed to be located in a room, according to this invention. The back rest of the chair contains Peltier cooling cells 62 powered from the outlet 63 over the converter box 64 by electric cords 65. The converter box 64 contains transformer 66, full wave rectifier 67, smoothing capacitor 68, and potentiometer 69 to regulate the d.c. voltage fed into the Peltier cell 62. The means of regulation 69 can be manual as well as programmed by a timer to reduce gradually the voltage to prevent back flow of heat from the hot plate to the cold plate when the cooling is terminated. Heat pipe 70 removes heat from the hot plate of the Peltier cells 62, and dissipates it through the fins 71.

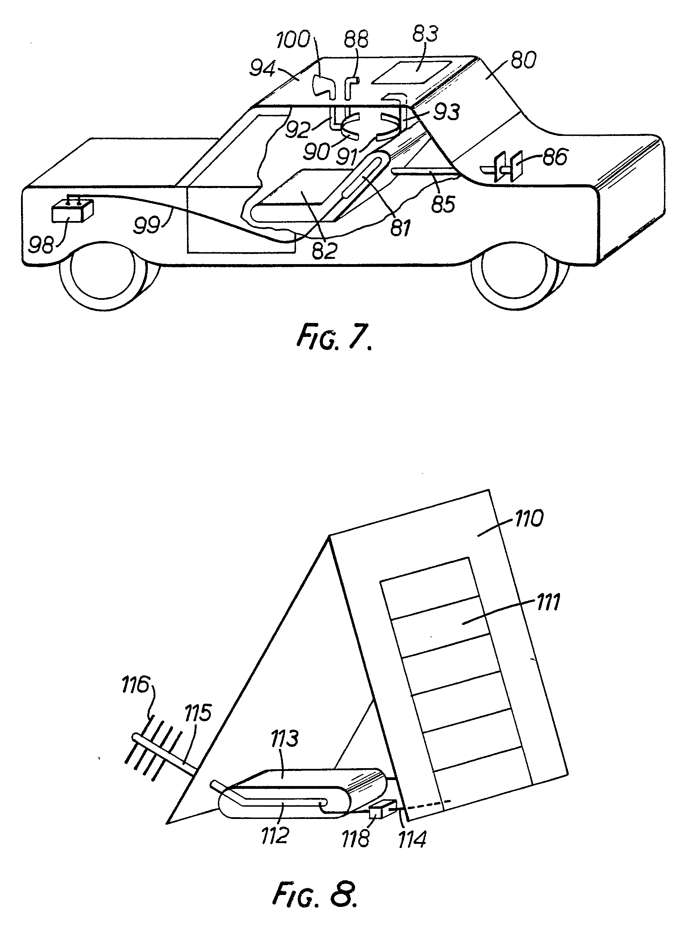

Figure 7 shows an automobile 80 containing Peltier cooling cells 81 in the driver's seat 82. The power for these Peltier cells comes from the car battery 98 through cable 99. The cold plates of the Peltier cells face the front of the back rest of seat 82. The heat pipe 85 conducts the heat from the hot plate of the Peltier cells to the heat dissipating fins 86 mounted at the outer surface of the chassis. The Peltier cooling rate in the 100 Watt range for the seats 61 in Figure 6 and 82 in Figure 7 demonstrates the tremendous power saving of this personalized Peltier cooling system as compared to cooling the entire enclosure, room, or car, as the case may be, by an air conditioning unit which typically consumes energy in the 1000 Watt range.

The automobile of Figure 7 also shows an arrangement for cooling the forehead by means of the Peltier cell collar 90 suspended from the car roof 94 by flexible tubing loop 92 through which air enters from the outside of the moving car through the inlet funnel 100, and exits through pipe 88.

The rear-of-the-neck cooling collar 91 is suspended from the roof 94 of the car by the bent flat heat pipe 93. The car roof 94 provides the heat sink for the hot plate of the Peltier cell 91 through metal heat pipe 93. The rigidity of the heat pipe 93 provides a head rest, while its flexibility prevents whiplash if the car should be rammed from the rear. It will be clear to those skilled in the art that position adjustment for the neck cooling can be provided, e.g. by two flat heat pipes 93 sliding against each other with clamping provisions. The construction of the Peltier cooled collars 90 and 91 is similar to that of Figure 3 except for the different means of dissipating heat from the hot plate. The personalized Peltier cooling systems 90 and 91 are powered from the solar cell array 83 located on the roof 94 of the car. Connecting cables have not been shown for the sake of clarity of illustration.

Figure 8 illustrates a tent 110 lined on one side by a solar cell array 111. The electric output of the solar cell array is connected by cable 114 to the Peltier cooling means 112 of sleeping bag 113 inside the tent. Heat pipe 115 conducts the heat of the hot plate of the Peltier cooling means 112 to heat dissipation fins 116 outside of the tent. Reversal of the current leading from the solar rack 111 to the Peltier cell 112 in sleeping bag 113 by means of the switch 118 causes heating of the sleeping bag with the cold of the cold plates removed by heat absorption through fin 116 from a cold ambient. It should be understood that the tent is normally closed, and that the front of the tent in Figure 8 is not shown solely for the purpose of clarity of representation of the inside fixture in the tent.

As there are many variations of the present invention, it should be understood that the invention is not limited to the specific embodiments here described, but encompasses all personalized cooling systems for warm blooded species, man or animal, using Peltier cells, which are defined by the following claims.

- 1 -

Claims:

1.      A personalized cooling system comprising at least one Peltier cell (10), said Peltier cell being powered by an electric power source (9, 29), means (26) to dissipate heat from the hot plate (6) of said Peltier cell, the cold plate (5) of said Peltier cell being adapted to be located in solid state thermal contact with the person to be cooled.

2.      The personalized cooling system of claim 1, wherein said Peltier cell (10) is attached to a garment (15) to be worn by said person.

3.      The personalized cooling system of claim 1, wherein said Peltier cell (62) is adapted to cool a fixture (61) to be located in contact with said person.

4.      The personalized cooling system of claim 3, wherein said fixture is a seat (61).

5.      The personalized cooling system of claim 2, wherein said garment is a head band (15).

6.      The personalized cooling system of claim 2, wherein said garment is a neck band (132).

7.      The personalized cooling system of claim 1, wherein said means to dissipate heat comprises cooling fins (26).

8.      The personalized cooling system of claim 7, wherein said cooling fins (26) are thermally connected to said hot plate (6) by a heat pipe (21).

9.    The personalized cooling system of claim 8, wherein said heat pipe (45) and cooling fins (46) are attached to a helmet (40).

10.    The personalized cooling system of claim 9, wherein at least one Peltier cell (41) is attached to said helmet (40).

11.    The personalized cooling system of claim 1, including a flat, curved heat pipe (20) adapted to be located between said cold plate (5) and said person to spread the cooling action of said Peltier cell (16) over a surface area of said person substantially exceeding the area of said cold plate (5).

12.    The personalized cooling system of claim 1, including a first heat pipe (20) attached to said cold plate (5) for spreading the cooling action of said cold plate over an area larger than that of said cold plate, a second heat pipe (21) attached to said hot plate (6) for spreading the heat of said hot plate over an area larger than the area of said hot plate, cooling fins (26) attached to said second heat pipe (21), said first heat pipe (20) being adapted to be located in intimate contact with a part of said person and shaped to conform with said part.

13.    The personalized cooling system of claim 12, including a thermally conducting resilient layer (33) adapted to be located between said first heat pipe (20) and said part of said person.

14.    The personalized cooling system of claim 1, including a layer (34) of metallized cloth adapted to be located between said cold plate (5) and said person to promote better thermal contact.

15. The personalized cooling system of claim 1, including means (14) for gradual reduction of the electric power for said Peltier cell (26).

16. The personalized cooling system of claim 15, wherein said means for gradual reduction of said electric power comprises a thermistor (14) in thermal contact with said hot plate (6) in combination with a switch (13) for inserting said thermistor into the electric circuit providing said power.

17. The personalized cooling system of claim 1, wherein said electric power source is a rechargeable battery (29).

18. The personalized cooling system of claim 17, wherein said battery (29) is adapted to be worn by said person.

19. The personalized cooling system of claim 17, including a portable array (35) of solar cells (9) for recharging said battery (29).

20. The personalized cooling system of claim 2, wherein said power source is a solar cell array (9).

21. The personalized cooling system of claim 20, wherein said solar cell array (9) is attached to a garment (19).

22. The personalized cooling system of claim 21, wherein said garment is a wide brimmed hat (19).

23. A personalized cooling system for cooling a fixture adapted to be located in close thermal contact

- 4 -

with a human in an enclosed environment, said personalized
cooling system including at least one Peltier cell (62),
means (64) to power said Peltier cell from an electric
power source (63), and means (71) to dissipate the heat
from the hot plate of said Peltier cell (62).

24.     The personalized cooling system of claim 23,
wherein said enclosed environment is a room, said fixture
is a chair (61) on which said human is to be seated, said
electric power source is an electric room outlet (63)
and said power means includes means (64) to transform
the a.c. voltage of said outlet to a d.c. voltage to be
applied to said Peltier cell (62).

25.     The personalized cooling system of claim 24,
wherein said means for heat dissipation comprises cooling fins (71) connected to said hot plate by a heat pipe
(70).

26.     The personalized cooling system of claim 23,
wherein said enclosed environment is a vehicle (80).

27.     The personalized cooling system of claim 26,
wherein said power source is a solar cell array (83)
carried on the outside of said vehicle.

28.     The personalized cooling system of claim 26,
wherein said means to dissipate heat comprises cooling
fins (86) attached to the chassis of said vehicle.

29.     The personalized cooling system of claim 26,
wherein said power source is the starter battery (98)
of said vehicle.

30.      A personalized cooling system for an occupant of an enclosed vehicle, said cooling system comprising at least one Peltier cell, means (90) to bring the cold plate of said Peltier cell into intimate thermal contact with a portion of the body of said occupant, and means for cooling the hot plate of said Peltier cell comprising a duct (100, 92, 88) through which an air stream is to be forced.

31.      The personalized cooling system of claim 30, wherein said air stream is to be generated by the motion of said vehicle.

32.      The personalized cooling system of claim 23, wherein said enclosed environment is a tent (110), said electric power source is a solar cell array (111) located outside of said tent, and said means to dissipate heat comprise cooling fins (116) positioned outside of said tent and connected by heat pipes (115) to said fixture (113) inside said tent.

33.      A personalized cooling system comprising at least one Peltier cell (112), one plate of said Peltier cell being adapted to be located in thermal contact with a person, the other plate of said Peltier cell being connected to fins (116) for heat exchange with the environment, said Peltier cell being connected to a d.c. power source (111), and means (118) to change the polarity of the current supplied to said Peltier cell from said power source, thereby providing heating to said person for one polarity of said current and cooling to said person for the other polarity of said current.

0050473

**FIG. 1.**
(PRIOR ART)

**FIG. 2.**

FIG. 3.

FIG. 4.

*FIG. 5.*

*FIG. 6.*

FIG. 7.

FIG. 8.